(19) Europäisches Patentamt
European Patent Office
Office européen des brevets

(11) Publication number: **0 380 264 A2**

# (12) EUROPEAN PATENT APPLICATION

(21) Application number: **90300625.2**

(22) Date of filing: **22.01.90**

(51) Int. Cl.⁵: **C07D 239/90, C07D 239/95, C07D 239/94, C07D 239/88**

(30) Priority: **23.01.89 US 300633**

(43) Date of publication of application:
**01.08.90 Bulletin 90/31**

(84) Designated Contracting States:
**AT BE CH DE DK ES FR GB GR IT LI NL SE**

(71) Applicant: **ELI LILLY AND COMPANY**
**Lilly Corporate Center**
**Indianapolis Indiana 46285(US)**

(72) Inventor: **Wright, Ian Glaisby**
**300 Meander Way**
**Greenwood, Indiana 46142(US)**

(74) Representative: **Hudson, Christopher Mark et al**
**Erl Wood Manor**
**Windlesham Surrey GU20 6PH(GB)**

## (54) Halogenation process.

(57) A process for preparing nitrogen-containing heterocyclic compounds that are chloro or bromo substituted at a ring position adjacent to the ring nitrogen atom which comprises reacting a hydroxy-substituted starting material with the kinetic form of a triphenylphosphite-halogen complex.

EP 0 380 264 A2

# HALOGENATION PROCESS

This invention provides a halogenation process for nitrogen-containing heterocyclic compounds.

In the past, the best method for synthesizing 4-chloroquinazoline and substituted 4-chloroquinazolines required refluxing a 4-hydroxyquinazoline with phosphorous pentachloride in phosphorous oxychloride. W.L.F. Armarego, J. Appl. Chem., 11 70 (1961). Isolation of the 4-chloroquinazoline required that it be separated from a large excess of reactive phosphorous halide compounds. This was difficult, hazardous, and unpleasant, because 4-chloroquinazoline is unstable, mutagenic, and smells bad.

U.S. Patent Appln. Serial No. 07/150,102 of Dreikorn et al. ( European Patent Appl. 89300657.7) discloses a new series of quinazoline fungicides, insecticides, and miticides. Among the compounds disclosed therein is, for example, 4-[2-[4-(t-butyl)-phenyl]ethoxy]quinazoline. Preparation of this compound can be accomplished by condensing 4-chloroquinazoline with 4-(t-butyl)benzeneethanol. A process for preparing 4-chloroquinazoline was desired in which it would not be necessary to isolate the 4-chloroquinazoline before reacting it with 4-(t-butyl)benzeneethanol. Using the known procedure for preparing the 4-chloroquinazoline, isolation is required, because otherwise the 4-(t-butyl)-benzeneethanol reacts preferentially with excess reactive phosphorous halides remaining from preparation of the 4-chloroquinazoline.

The present invention provides a new process for preparing 4-chloroquinazoline, and other halogenated nitrogen-containing heterocyclic compounds. One of the advantages of this new process is that a 4-chloroquinazoline prepared thereby need not be isolated before it is reacted with, for example, an alcohol, to produce one of the compounds of the above identified Dreikorn et al. application.

More specifically, the present invention provides a process for preparing nitrogen-containing heterocyclic compounds that are chloro or bromo substituted at a ring position adjacent to the ring nitrogen atom, which comprises reacting a nitrogen containing heterocyclic starting material that is hydroxy-substituted at a ring position adjacent to the ring nitrogen atom with the kinetic form of a triphenylphosphite-halogen complex having the formula:

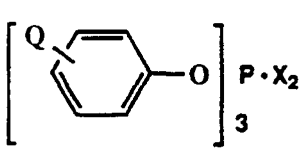

(1)

where Q is H, halo, ($C_1$-$C_4$) alkyl or ($C_1$-$C_4$) alkoxy, and X is Cl or Br.

The triphenylphosphite-halogen complex halogenating reagents useful in practicing this invention are described in U.S. Patent No. 4,230,644 (incorporated by reference herein), where their use in converting alcohols to alkyl halides and amides to imino chlorides is described. The prior art did not suggest use of these halogenating reagents to halogenate nitrogen-containing heterocyclic compounds.

The triphenylphosphite-halogen reagent of formula (1) is prepared by reacting chlorine or bromine with a suitable triphenylphosphite in a substantially anhydrous inert organic solvent, such as a hydrocarbon, or halogenated hydrocarbon, at a temperature below 30° C, preferably -15° C to 0° C. The triphenylphosphite-halogen reagent is unstable and converts on standing to a less reactive "thermodynamic" form. The "kinetic" form can be stabilized in solution by adding up to 1 mole of a tertiary amine base, such as pyridine, to the reaction mixture, and by operating at lower temperatures, e.g. less than about -15° C.

To minimize the opportunity for equilibration to the less reactive thermodynamic product, the halogenating reagents may be prepared immediately before they are utilized, or preferably are prepared in the presence of the hydroxy-substituted nitrogen-containing heterocycle so that reaction is immediate. Typically, the halogen is added to a mixture of the triphenylphosphite, pyridine, and hydroxy-substituted nitrogen-containing heterocycle in a suitable solvent.

Preferably, 1 to 1.3 equivalents of halogenating reagent of formula (1) are used per equivalent of hydroxy-substituted starting material. Following preparation of the halogenated nitrogen-containing heterocycle, excess halogenating reagent may be quenched by adding a small amount of water to the reaction mixture. An alcohol or amine may then be condensed with the halogenated compound without isolation.

The following are examples of nitrogen-containing heterocyclic starting materials that may be halogenated using the process of this invention:
4-hydroxyquinazoline,
2-hydroxyquinazoline,
2-hydroxypyrrole,
4-hydroxyimidazole,
3-hydroxypyrazole,
2-hydroxypyridine,
2-hydroxypyrazine,
2-hydroxypyrimidine,
4-hydroxypyrimidine,

3-hydroxypyridazine,
3-hydroxyindolizine,
5-hydroxyindolizine,
1-hydroxyisoindole,
3-hydroxyisoindole,
2-hydroxyindole,
6-hydroxypurine,
3-hydroxyisoquinoline,
1-hydroxyisoquinoline,
2-hydroxyquinoline,
4-hydroxyphthalazine,
1-hydroxyphthalazine,
7-hydroxy-1,8-naphthyridine,
2-hydroxy-1,8-naphthyridine,
3-hydroxycinnoline,
4-hydroxypteridine,
6-hydroxyphenanthridine,
3-hydroxyisothiazole,
3-hydroxyisoxazole,
4-hydroxythieno[2,3-d]pyrimidine,
4-hydroxythieno[3,2-d]pyrimidine,
4-hydroxyfuro[2,3-d]pyrimidine,
4-hydroxyfuro[3,2-d]pyrimidine.

Particularly preferred hydroxy-substituted nitrogen-containing heterocyclic starting materials are 4-hydroxyquinazolines of the formula (2)

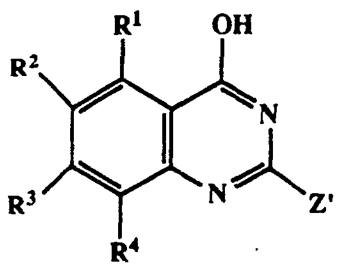

(2)

wherein $R^1$ to $R^4$ are independently H, halo, ($C_1$-$C_4$) alkyl, branched ($C_3$-$C_4$) alkyl, halo ($C_1$-$C_4$) alkyl, halo ($C_1$-$C_4$) alkoxy, ($C_1$-$C_4$) alkoxy, halo ($C_1$-$C_4$) alkylthio, ($C_1$-$C_4$) alkylthio, or $NO_2$, at least two of $R^1$ to $R^4$ being H; and $Z'$ is H, Cl, $CH_3$, or $OCH_3$.

Another preferred aspect of the invention comprises a process for preparing a compound of formula (3)

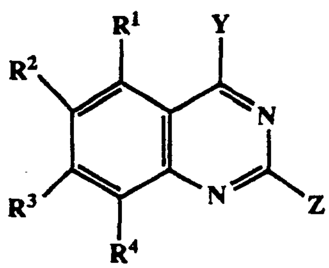

(3)

wherein:

$R^1$ to $R^4$ are independently H, halo, ($C_1$-$C_4$) alkyl, branched ($C_3$-$C_4$) alkyl, halo ($C_1$-$C_4$) alkyl, halo ($C_1$-$C_4$) alkoxy, ($C_1$-$C_4$) alkoxy, halo ($C_1$-$C_4$) alkylthio, ($C_1$-$C_4$) alkylthio, or $NO_2$, at least two of $R^1$ to $R^4$ being H;

Y is O-Alk, O-W-Ar; or $NR^7$-W-Ar;

Z is H, Cl, $OCH_3$, $CH_3$, $CCl_3$, or -$NR^7$-W-Ar, provided that Z can be -$NR^7$-W-Ar only if Y is -$NR^7$-W-Ar;

$R^7$ is H, or ($C_1$-$C_4$) alkyl;

W is an alkylene chain 2 to 8 carbon atoms long, that optionally includes an O, S, SO, $SO_2$, or $NR^7$ group, or includes a saturated or unsaturated carbocyclic ring comprising three to seven carbon atoms, or is substituted with ($C_1$-$C_3$) alkyl, ($C_2$-$C_4$) alkenyl, phenyl, ($C_3$-$C_8$) cycloalkyl, halo, or acetyl; and

Ar is
imidazolyl;
indolyl;
thienyl, optionally substituted with $CH_3$ or Cl;
thiazolyl;
1,3-benzodioxolyl;
fluorenyl;
cyclopentyl;
1-methylcyclopentyl;
cyclohexyl (hexahydrophenyl),
cyclohexenyl (tetrahydrophenyl),
naphthyl,
dihydronaphthyl,
tetrahydronaphthyl,
decahydronaphthyl;
pyridyl;
or a group of the formula (4):

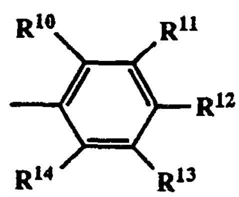

(4)

where

$R^{10}$ to $R^{14}$ are independently H, halo, I, ($C_1$-$C_{10}$) alkyl, branched ($C_3$-$C_6$) alkyl, halo ($C_1$-$C_4$) alkyl, ($C_1$-$C_4$) alkoxy, halo ($C_1$-$C_4$) alkoxy, phenoxy, substituted phenoxy, phenyl, substituted phenyl, phenylthio, substituted phenylthio, $NO_2$, acetoxy, CN, $SiR^{15}R^{16}R^{17}$, $OSiR^{15}R^{16}R^{17}$, where $R^{15}$, $R^{16}$, and $R^{17}$ are independently $C_1$-$C_4$ alkyl, $C_3$-$C_4$ branched alkyl, phenyl, or substituted phenyl, at least two of $R^{10}$ to $R^{14}$ being H;

Alk is a ($C_2$-$C_{18}$) saturated or unsaturated hydrocarbon chain, straight chain or branched, optionally substituted with halo, halo ($C_1$-$C_4$) alkoxy, ($C_3$-$C_8$) cycloalkyl, hydroxy, or ($C_1$-$C_4$) acyl;

or an acid addition salt of a compound of formula (1);

which comprises:

(a) reacting a 4-hydroxyquinazoline of formula (2)

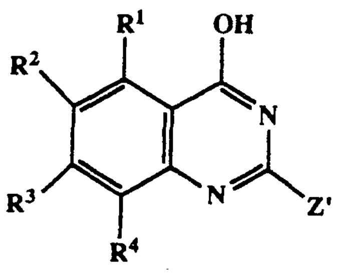

(2)

wherein Z′ is H, Cl, $CH_3$, or $OCH_3$ and $R^1$, $R^2$, $R^3$, and $R^4$ are as defined above, with 1 to 1.3 equivalents of a halogenating reagent of the formula (1)

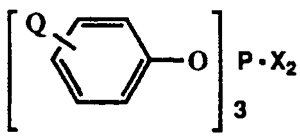

(1)

wherein Q is H, halo, ($C_1$-$C_4$) alkyl, or ($C_1$-$C_4$) alkoxy and X′ is Cl or Br, in an inert organic solvent at a temperature below 30° C, and

(b) without isolation of the 4-chloroquinazoline produced in step (a), reacting it with an alcohol of the formula (5a) or (5b)

HO-Alk (5a), or

HO-W-Ar (5b),

where Alk, W, and Ar are as previously defined, or with an amine of the formula (6)

$HNR^7$-W-Ar (6)

where $R^7$, W, and Ar are as previously defined.

Example 1

4-[2-[4-(t-Butyl)phenyl]ethoxy]quinazoline

To 54 L of methylene chloride, and 1.8 L of pyridine, 6.5 Kg of 4-hydroxyquinazoline was added, and the mixture was cooled to -5 to 10° C. To this mixture 15.7 Kg of triphenylphosphite was added. Then 3.67 Kg of chlorine gas was bubbled in over a three hour period while the temperature of the mixture was maintained in the range of 0 to 10° C, to produce 4-chloroquinazoline hydrochloride. After stirring the mixture an additional hour, 105 mL of water was added to quench excess halogenating reagent. Then 9.7 Kg of 4-(t-butyl)-benzeneethanol was added over a 15 minute period while the mixture was maintained at 10 to 20° C. The mixture was heated to reflux (40-45° C) and held at reflux for three hours. After vacuum distilling the contents of the reaction to minimize volume, 56 L of toluene was added. The contents of the reaction were again vacuum distilled, 56 L of heptane were added, and the mixture was cooled to 30-35° C. The HCl salt of the desired product was isolated by filtration, and washed with toluene/heptane.

The free base was obtained by combining the wet cake of the HCl salt with 58 L acetone and 6.5 L of triethylamine. After heating the mixture to 25-30° C the contents of the reaction were vacuum distilled to a volume of 45 L, and then 70 L of water were added. The mixture was cooled to 5-10° C over 45 minutes, and the title product was then isolated by filtration. Yield: 11.5 Kg (85%).

As used herein the terms "substituted phenyl", "substituted phenoxy", and "substituted phenylthio" refer to groups substituted with up to three groups selected from F, Cl, Br, I, ($C_1$-$C_{10}$) alkyl, branched ($C_3$-$C_6$) alkyl, halo ($C_1$-$C_4$) alkyl, ($C_1$-$C_4$) alkoxy, halo ($C_1$-$C_4$) alkoxy, phenoxy, phenyl, $NO_2$, CN, ($C_1$-$C_4$) alkanoyloxy, or benzyloxy.

## Claims

1. A process for preparing nitrogen-containing heterocyclic compounds that are chloro or bromo substituted at a ring position adjacent to the ring nitrogen atom, which comprises: reacting a nitrogen containing heterocyclic starting material that is hydroxy-substituted at a ring position adjacent to the ring nitrogen atom with the kinetic form of a triphenylphosphite-halogen complex having the formula:

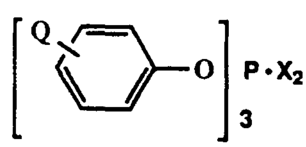

(1)

where Q is H, halo, ($C_1$-$C_4$) alkyl or ($C_1$-$C_4$) alkoxy, and X is Cl or Br.

2. The process of claim 1 wherein the triphenylphosphite-halogen complex is prepared in the presence of the hydroxy-substituted heterocyclic starting material.

3. The process of claim 1 carried out in the presence of a tertiary amine base.

4. The process of claim 1 wherein 1 to 1.3 equivalents of triphenylphosphite-halogen complex are used per mole of hydroxy-substituted starting material.

5. The process of claim 1 wherein the nitrogen containing heterocyclic starting material is an optionally substituted 4-hydroxyquinazoline.

6. The process of claim 5 wherein the optionally substituted 4-hydroxyquinazoline starting material is of the formula

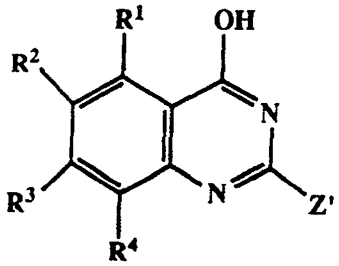

(2)

wherein $R^1$ to $R^4$ are independently H, halo, ($C_1$-$C_4$) alkyl, branched ($C_3$-$C_4$) alkyl, halo ($C_1$-$C_4$) alkyl, halo ($C_1$-$C_4$) alkoxy, ($C_1$-$C_4$) alkoxy, halo ($C_1$-$C_4$) alkylthio, ($C_1$-$C_4$) alkylthio, or $NO_2$, at least two of $R^1$ to $R^4$ being H; and Z′ is H, Cl, $CH_3$, or $OCH_3$.

7. The process of claim 6 wherein the starting material is 4-hydroxyquinazoline.

8. A process for preparing a compound of formula (3)

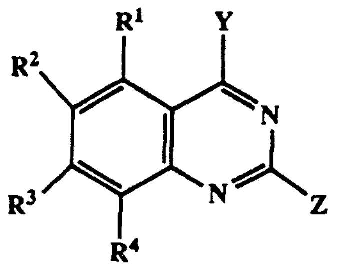

(3)

wherein:
$R^1$ to $R^4$ are independently H, halo, ($C_1$-$C_4$) alkyl, branched ($C_3$-$C_4$) alkyl, halo ($C_1$-$C_4$) alkyl, halo ($C_1$-$C_4$) alkoxy, ($C_1$-$C_4$) alkoxy, halo ($C_1$-$C_4$) alkylthio, ($C_1$-$C_4$) alkylthio, or $NO_2$, at least two of $R^1$ to $R^4$ being H;
Y is O-Alk, O-W-Ar, or $NR^7$-W-Ar;
Z is H, Cl, $OCH_3$, $CH_3$, $CCl_3$, or -$NR^7$-W-Ar, provided that Z can be -$NR^7$-W-Ar only if Y is -$NR^7$-W-Ar;
$R^7$ is H, or ($C_1$-$C_4$) alkyl;
W is an alkylene chain 2 to 8 carbon atoms long, that optionally includes an O, S, SO, $SO_2$, or $NR^7$ group, or includes a saturated or unsaturated carbocyclic ring comprising three to seven carbon atoms, or is substituted with ($C_1$-$C_3$) alkyl, ($C_2$-$C_4$) alkenyl, phenyl, ($C_3$-$C_8$) cycloalkyl, halo, or acetyl; and
Ar is
imidazolyl;
indolyl;
thienyl, optionally substituted with $CH_3$ or Cl;
thiazolyl;
1,3-benzodioxolyl;
fluorenyl;
cyclopentyl;
1-methylcyclopentyl;
cyclohexyl (hexahydrophenyl),
cyclohexenyl (tetrahydrophenyl),
naphthyl,
dihydronaphthyl,
tetrahydronaphthyl,
decahydronaphthyl;
pyridyl;
or a group of the formula (4):

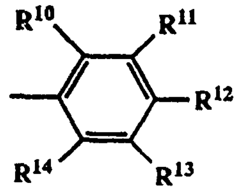

(4)

where
$R^{10}$ to $R^{14}$ are independently H, halo, I, ($C_1$-$C_{10}$) alkyl, branched ($C_3$-$C_6$) alkyl, halo ($C_1$-$C_4$) alkyl, ($C_1$-$C_4$) alkoxy, halo ($C_1$-$C_4$) alkoxy, phenoxy, substituted phenoxy, phenyl, substituted phenyl, phenylthio, substituted phenylthio, $NO_2$, acetoxy, CN, $SiR^{15}R^{16}R^{17}$, $OSiR^{15}R^{16}R^{17}$, where $R^{15}$, $R^{16}$, and $R^{17}$ are independently $C_1$-$C_4$ alkyl, $C_3$-$C_4$ branched alkyl, phenyl, or substituted phenyl, at least two of $R^{10}$ to $R^{14}$ being H;
Alk is a ($C_2$-$C_{18}$) saturated or unsaturated hydrocarbon chain, straight chain or branched, optionally substituted with halo, halo ($C_1$-$C_4$) alkoxy, ($C_3$-$C_8$) cycloalkyl, hydroxy, or ($C_1$-$C_4$) acyl;
or an acid addition salt of a compound of formula (1);
which comprises:

(a) reacting a 4-hydroxyquinazoline of formula (2)

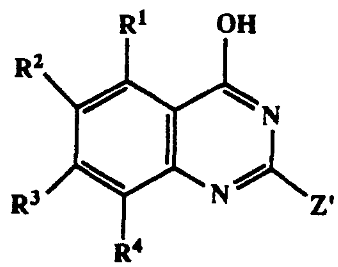

(2)

wherein Z′ is H, Cl, $CH_3$, or $OCH_3$ and $R^1$, $R^2$, $R^3$, and $R^4$ are as defined above, with 1 to 1.3 equivalents of a halogenating reagent of the formula (1)

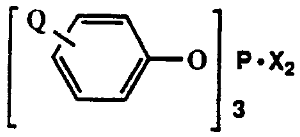

(1)

wherein Q is H, halo, ($C_1$-$C_4$) alkyl, or ($C_1$-$C_4$) alkoxy and X′ is Cl or Br, in an inert organic solvent at a temperature below 30° C, and

(b) without isolation of the 4-chloroquinazoline produced in step (a), reacting it with an alcohol of the formula (5a) or (5b)

HO-Alk (5a), or

HO-W-Ar (5b),

where Alk, W, and Ar are as previously defined, or with an amine of the formula (6)

$HNR^7$-W-Ar (6)

where $R^7$, W, and Ar are as previously defined.

9. A process of claim 8 wherein the starting material of formula (2) is 4-hydroxyquinazoline.

10. A process of claim 9 wherein step (b) comprises reacting the 4-chloroquinazoline with 4-(*t*-butyl)benzeneethanol.